# EUROPEAN PATENT SPECIFICATION

(11) **EP 1 042 270 B1**
(45) Date of publication and mention of the grant of the patent: **09.04.2003**
(21) Application number: 98960039.0
(22) Date of filing: 16.12.1998
(51) Int. Cl.: C07C 67/303, C07C 69/34, C07C 69/608

(54) **ASYMMETRIC HYDROGENATION**
ASYMMETRISCHE HYDRIERUNG
HYDROGENATION ASYMETRIQUE

(30) Priority: 17.12.1997 GB 9726679; 17.02.1998 GB 9803356; 09.04.1998 GB 9807888
(43) Date of publication of application: 11.10.2000
(73) Proprietor: Chirotech Technology Limited, Cambridge CB4 0WG (GB)
(72) Inventor: BURK, Mark, Joseph, Chirotech Technology Limited, Milton Road, Cambridge CB4 0WG (GB); BEINEWALD, Frank, F-78000 Versailles (FR); FOX, Martin, Edward, Chirotech Technology Limited, Milton Road, Cambridge CB4 0WG (GB); ZANOTTI-GEROSA, Antonio, Milton Road, Cambridge CB4 0WG (GB)
(74) Representative: Perry, Robert Edward
(86) International application number: GB9803784
(87) International publication number: WO99031041

(56) References cited:
- WO-A-97/18894
- US-A- 4 939 288
- GEORGE BASHIARDES ET AL.: "Chiral Succinate Enolate Equivalents for the Asymmetric Synthesis of alpha-Alkyl Succinic Acid Derivatives" JOURNAL OF THE CHEMICAL SOCIETY, PERKIN TRANSACTIONS 1., no. 6, June 1989, pages 1162-1164, XP002076527 LETCHWORTH GB

## Description

This invention relates to processes suitable for the large scale preparation of enantiomerically enriched 2-substituted succinic acid derivatives. In particular, it relates to asymmetric hydrogenation of isomeric mixtures of (*E*)- and (*Z*)-itaconate derivatives, as well as β,β-disubstituted itaconate derivatives, using a transition metal catalyst complex.

### Background of the Invention

Enantiomerically enriched 2-substituted succinic acids (see formulae **2a** and **2b,** below) have recently attracted interest as useful chiral building blocks and peptidomimetics in the design of pharmaceuticals, flavours and fragrances, and agrochemicals with improved properties. For example, the utility of 2-substituted succinic acid derivatives has been amply demonstrated through the synthesis of a range of new potent orally bioavailable drugs [J.J. Talley et al., in *Catalysis of Organic Reactions*, J.R. Kosak, T.A. Johnson (eds.), Marcel Dekker, Inc. (**1994**) Chapter 6; and H. Jendralla, *Synthesis* (**1994**) 494].

Chiral succinates can be prepared simply (e:g., via Stobbe condensation) from unsubstituted succinic esters and aldehydes or ketones, followed by asymmetric hydrogenation of the intermediate β-substituted itaconate derivatives. The latter reaction may be represented as

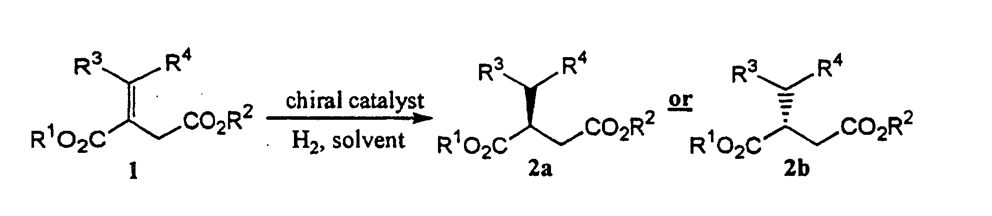

The unsubstituted parent substrate, itaconic acid (1, R¹=R²=R³=R⁴=H), or its sodium salt, can be enantioselectively hydrogenated to 2-methylsuccinic acid with rhodium catalysts bearing the chiral ligand N-acyl-3,3 '-bis(diphenylphosphino)pyrrolidine (BPPM) in up to 92% enantiomeric excess (ee) [I. Ojima et al., *Chem. Lett.,* **1978,** 567; I. Ojima et al., *Chem. Lett.,* **1978,** 1145; K. Achiwa, *Tetrahedron Lett.,* **1978,** 1475]. A rhodium catalyst bearing the chiral diphosphine DIPAMP affords 2-methylsuccinate in up to 88 % ee [W. C. Christofel, B. D. Vineyard, *J. Am. Chem. Soc.* **1979,** *101,* 4406; and US-A-4,939,288]. Similar results have been obtained with a ruthenium catalyst containing the chiral diphosphine ligand BINAP [H. Kawano et al., *Tetrahedron Lett.,* **1987,** *28,* 1905]. Rhodium catalysts bearing modified DIOP ligands provide 2-methylsuccinic acid derivatives with variable enantioselectivities, between 7 and 91% ee. In these latter reactions, the ee value is very dependant on the rhodium catalyst precursor and whether the free acid or the ester is used [T. Morimoto et al., *Tetrahedron Lett.,* **1989,** *30,* 735]. Better results have been reported with a neutral rhodium catalyst of the chiral diphosphine 2,2'-bis(dicyclohexylphosphino)-6,6'-dimethyl-1,1 '-biphenyl (BICHEP), whereby dimethylitaconate was hydrogenated in 99% ee [T. Chiba et al., *Tetrahedron Lett*., **1991,** *32,* 4745].

In contrast to the success achieved with unsubstituted itaconate derivatives, asymmetric hydrogenation of β-substituted itaconic acid derivatives of general structure **3** and **4** (R³ ≠ H) has been more challenging; relatively few reports of high enantioselectivity (over 90% ee) have appeared. The curtailed effectiveness of known asymmetric catalysts for the hydrogenation of β-substituted itaconic acids derivatives 3 and/or **4** is particularly apparent when R³ is an alkyl group, in which case no enantioselectivities above 90% ee have been reported.

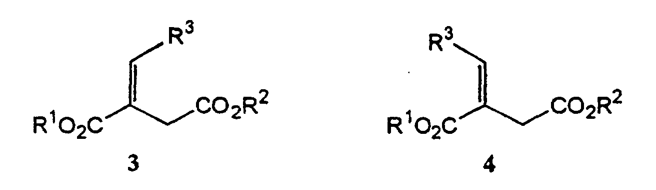

It should be noted that enantiomerically pure compounds are required for many applications in, for example, the pharmaceutical industry. Consequently, providing enantiomeric purity is the ultimate objective of an asymmetric process, and achieving high enantioselectivity in a transformation of the type described herein is crucial from a process standpoint. 90% ee is often selected as a lower acceptable limit, because compounds often may be purified to enantiomeric purity through recrystallisation when the initial value is above 90% ee. Enantiomeric excesses lower than 90% ee become increasingly more difficult to purify.

A major complication encountered with β-substituted itaconic acid derivatives is that they frequently are synthesised as a mixture of *E* and *Z* geometric isomers, i.e. formulae **3** and **4** respectively. This presents serious difficulties in subsequent hydrogenation reactions, since the different geometric isomers typically are reduced with vastly different rates and enantioselectivities. Accordingly, it has been necessary to separate the E- and Z-isomers (**3** and **4**) prior to the hydrogenation reaction. From a process standpoint, this is a wasteful, time-consuming, and yield-limiting feature.

Several reports have disclosed the ability to hydrogenate pure β-substituted (*E*)-itaconate derivatives (**3**). Even when the substrates are *E*-pure, however, high enantioselectivities have been limited to hydrogenation of β-arylitaconate derivatives (**3**, R³ = aromatic ring such as phenyl or naphthyl). Isomerically pure (*E*)-β-phenylitaconate derivatives have been hydrogenated with a DIPAMP-Rh catalyst in up to 97% ee [US-A-4,939,288]. The same substrates were hydrogenated with a Ru-BINAP catalyst in up to 90% ee [H. Kawano et al., *Tetrahedron Lett.,* **1987,** *28,* 1905]. A rhodium complex bearing a modified DIOP ligand allowed hydrogenation of several (*E*)-β-arylitaconate derivatives with enantioselectivities up to 96% ee [T. Morimoto et al., *Tetrahedron Lett*., **1989,** 30, 735].

Despite these impressive results, relatively poor enantioselectivities have been reported in the hydrogenation of (*E*)-pure β-alkylitaconate derivatives (**3**, R³ = alkyl). The DIPAMP-Rh catalyst noted above provided 2-isobutylsuccinate in only 76 % ee through hydrogenation of (*E*)-substrate **3** (R³ = CH(CH₃)₂)[US-A-4,939,288]. Importantly, it is indicated that the (*Z*)-isomer of this substrate (i.e., **4,** R³ = CH(CH₃)₂) is not a suitable substrate for this reaction and must be separated from (*E*)-substrate prior to hydrogenation. The low enantioselectivity achieved with this particular substrate using known catalysts is regrettable since the product succinate **2** (R³ = CH(CH₃)₂) serves as a critical component of numerous new drug candidates.

Itaconate derivatives that possess two substituents in the β-position (β,β-disubstituted itaconates of formula **1** where R³,R⁴≠H) have thus far proven impossible to hydrogenate with high enantioselectivities and high rates. The only reported example of this type revealed that dimethyl β,β-dimethylitaconate may be hydrogenated with a Rh-TRAP catalyst system with the highest enantioselectivities being 78% ee [R. Kuwano et al., *Tetrahedron: Asymmetry*, **1995,** *6*, 2521].

### Summary of the Invention

One aspect ofthe present invention is based on the discovery that a broad variety of different itaconate derivatives can be readily hydrogenated using certain chiral mono and diphosphine-transition metal catalysts, to afford highly enantiomerically enriched **2**-substituted succinates. Another aspect of the present invention lies in the realisation that, when R³ and/or R⁴ has an α-H atom, an isomerised product is formed; this undesired product can be removed, once its presence is appreciated.

A further aspect of the present invention is based on the discovery that an efficient and high yielding preparation of an enantiomerically enriched 2-substituted succinic acid derivative (2), by asymmetric hydrogenation is the presence of a transition metal complex of a chiral phosphine, is facilitated by use of particular salt forms of the hydrogenation substrate.

Important characteristics of the present invention include: (i) the ability to hydrogenate β-alkyl-substituted itaconate derivatives with very high enantioselectivities, (ii) the ability to hydrogenate an isomeric mixture of (*E*)- and (*Z*)-itaconate derivatives with very high enantioselectivities, (iii) the ability to hydrogenate β,β-disubstituted itaconate derivatives with high enantioselectivities, (iv) the ability to hydrogenate specifically itaconate substrates in the presence of an isomerised (deconjugated) analogue of the substrate formed during substrate synthesis, (v) a convenient procedure for separation of the succinate products and remaining isomerised substrate leading to facile purification of the products, and (vi) an efficacious overall process encompassing the above features for the synthesis of enantiomerically pure 2-substituted succinate derivatives. Either enantiomer of the 2-substituted succinate derivative may be obtained by the method described herein.

In addition, for certain hydrogenation substrates, use of salt forms can have a number of advantages. Firstly, formation and isolation of a salt form may provide a convenient means of effecting substrate purification prior to hydrogenation, should this be required. Secondly, at a given molar ratio of substrate to catalyst (S/C ratio) and reaction time, a higher substrate conversion and/or higher enantioselectivity can be achieved. Thirdly, high reaction rates allow reactions to be performed at low temperatures, e.g. 0°C, whereby higher product enantiopurity is observed.

### Description of the Invention

Both β-substituted and β,β-disubstituted itaconate derivatives may be prepared most readily by Stobbe condensation between dialkyl succinates **5** and aldehydes (6, R³ = H) or ketones (**6**, R³,R⁴ ≠ H) in the presence of a base. A review article describing the Stobbe condensation may be found in *Org. React*., **1951,** *6*, 1-73. In general, the Stobbe condensation leads to a mixture of *E* and *Z* isomeric itaconate derivatives **7** and **8,** especially in the case where R³ and/or R⁴ are alkyl groups.

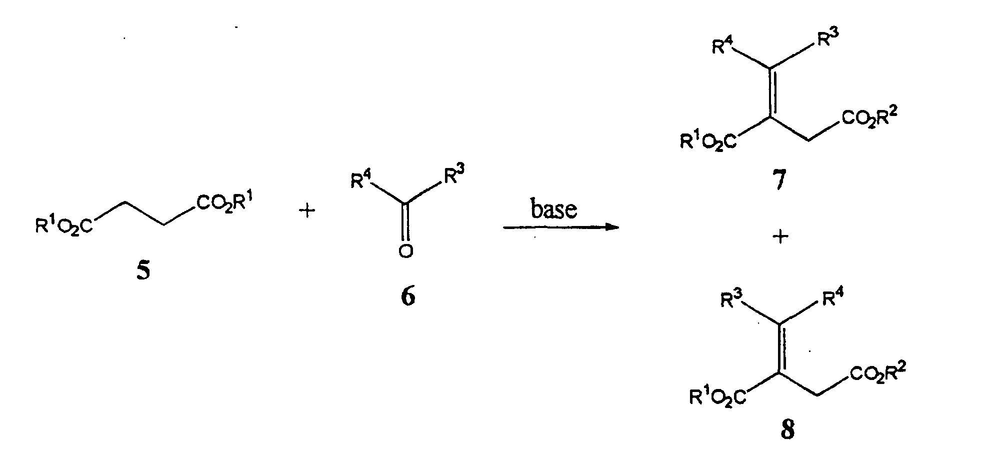

Typically, in products of the Stobbe condensation, R¹ is an esterifying group and R² is H. Such compounds are preferred starting materials for use in this invention.

Moreover, if R³ or R⁴ in aldehyde or ketone **6** contains a C-H group adjacent to the carbonyl (C=O) function, e.g. if R⁴ is CHR⁵R⁶ where R⁵ and R⁶ are each H or any other group consistent with the definition of R⁴, then a significant amount of isomerised (deconjugated) compound **9** may also be formed. In some cases, e.g. depending on the catalyst, this can be the major product of the Stobbe condensation reaction. The presence of isomerised compound **9** in the mixture is potentially detrimental, since any hydrogenation of this material will afford racemic succinate product.

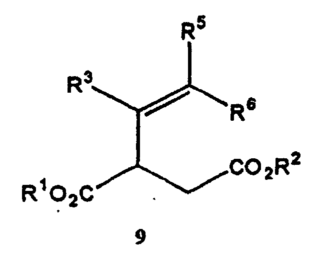

In accordance with the present invention, a mixture of Stobbe products composed of *E* and Z isomers **7** and **8,** along with regioisomeric deconjugated compound **9,** may be employed without any special purification in the hydrogenation reaction in which only the itaconate substrates **7** and **8** are reduced to desired succinate product (**2a** or **2b**) with very high enantioselectivity. This stands in contrast to all previous processes in which only purified (*E*)-itaconate substrates could be employed in the hydrogenation procedure.

As a further aspect of the present invention, it has been discovered that a Stobbe product mixture of a β,β-disubstituted itaconate derivative (e.g., 7, **R**^{**3**}**,R**^{**4**} ≠ H) and regioisomer **9,** can be treated such that only the itaconate derivative is hydrogenated to the desired succinate product (**2a** or **2b**), with high enantioselectivity. No other catalyst system previously has been shown to allow asymmetric hydrogenation of β,β-disubstituted itaconate derivatives with high enantioselectivity.

Suitable substrates for the hydrogenation process outlined above are ofthe general structure **7** or **8,** or a mixture thereof, wherein R¹, R², R³ and R⁴ can be independently H or an organic group of up to 30 C atoms, or R¹ and R² are independently a cation, or R³ and R⁴ are joined to form a ring, provided that at least one of R³ and R⁴ is not H. In one embodiment, the invention provides an improved procedure in the case where one of R³ and R⁴ is H; typically, the other is C₁₋₂₀ alkyl or aralkyl. The fact that β,β-disubstituted itaconates can be effectively hydrogenated in this process means also that R³ and R⁴ may each be an organic group of up to 30 C atoms, e.g. C₁₋₂₀ alkyl or aralkyl, and preferably the same, or may be linked to form a ring, e.g. a saturated carbocyclic ring. R¹ is preferably C₁₋₁₀ alkyl or aralkyl, and R² is preferably H (or a cation).

If a salt of the precursor is used, it may be a salt form of either itaconate derivative which may be either β-substituted or β,β-disubstituted. In this case, R² may represent a metal, e.g. alkali metal, or other cation. Typically, this is formed *in situ,* by introducing a strong base such as a metal alkoxide, e.g. NaOMe. This may be used in a substoichiometric amount, e.g. a catalytic quantity.

Alternatively, the salt may be formed with, for example, a counterion YH⁺ such as that derived from an amine Y or a phosphine Y. Primary C₁₋₁₀ alkylamines and cycloalkylamines are preferred, in particular, *tert*-butylamine. Tertiary amines such as triethylamine may also be used.

Especially when an amine or phosphine salt is used, it is usually isolated prior to use in the process, but alternatively may be generated *in situ.* Isolation of the precursor salt can be advantageous as a means of effecting substrate purification, usually by crystallisation, e.g. to remove any regioisomeric contaminants. However, this step is not always necessary, especially when the Stobbe condensation is carried out under carefully controlled conditions where regioisomeric contaminants are not formed, e.g. at a temperature of around 5°C rather than at normal room temperature.

Temperature effects may also be noted in the process ofthe present invention, with a lowering of reaction temperature resulting in improved enantioselectivities for certain substrates, e.g. when R³/R⁴ is a cyclic group, or if the precursor is an amine or phosphine salt. Especially in such cases, the reaction temperature may be less than 10°C, and is preferably -25 to +5°C.

Catalysts that are suitable for the novel asymmetric hydrogenation process comprise a transition metal complexed to an appropriate chiral phosphine ligand. Preferably, the ligand is a monophosphine or diphosphine ligand which may be used in either enantiomeric form. The preferred transitional metal is rhodium; others that may be used include ruthenium and iridium.

Preferred phosphines are those incorporating an appropriately substituted phosphorus heterocycle of general structure **10**, where n is zero or an integer 1 to 6, and where the carbocyclic framework of **10** is substituted with one or more R substituents such that the structure **10** is a chiral entity, and where the R substituent is an organic group of up to 20 C atoms, typically a C₁₋₁₀ linear or branched hydrocarbon substituent, but which also may contain heteroatoms. In the case where more than one R substituent is present in the structure **10**, these R substituents may be the same or different, and may be joined to form ring systems fused with the parent carbocyclic framework illustrated for **10**. Monophosphines containing the phosphorus heterocyclic unit 10 take the general structure **11**, where R' is an organic group of up to 20 C atoms. Alternatively, two phosphorus heterocycles of structure 10 may be tethered with a linking unit to form a diphosphine of general structure **12**, where the linking unit is an organic group of up to 30 C atoms, linear, branched or cyclic, hydrocarbon or heteroatomic in nature.

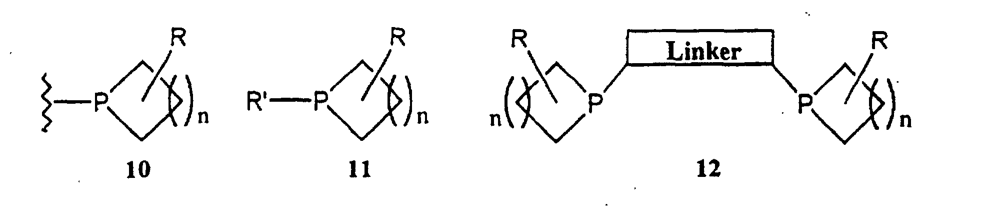

Examples of these ligands encompass 2,4-disubstituted phosphetanes **13,** e.g. as disclosed in WO-A-9802445, as well as the DuPHOS [US-A-5171892] and BPE [US-A-5008547] series of bisphospholanes, **14** and **15**, respectively. The latter ligands constitute the most preferred class of diphosphines for the asymmetric hydrogenation process described herein.

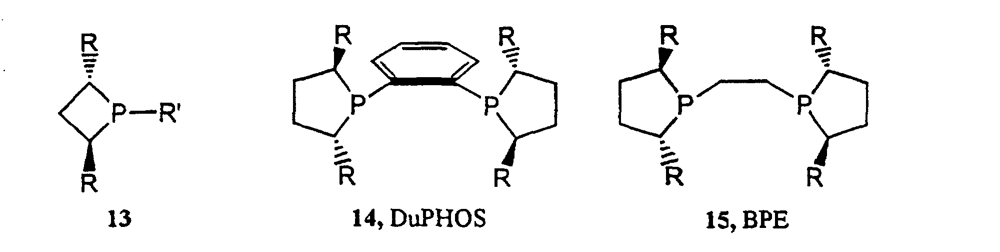

The possession of a series of homologous ligands of types **11-15** which are substituted with a range of different R groups is crucial for success in asymmetric hydrogenations since it is difficult to predict which catalyst will hydrogenate a particular substrate type with high selectivity. For a given substrate, enantioselectivities may be dependent upon the nature of the R-substituent attached to the carbocyclic ring of the DuPHOS, BPE or other ligand (as can be seen from Table 1, below). Typically, a range of ligand-metal complexes may be screened, in order to identify the optimum catalyst for a given transformation, although such screening is readily done by one of ordinary skill in the art, if necessary with reference to the guidance provided herein. The appropriate complex may change, from substrate type to substrate type: rhodium complexes containing certain DuPHOS and BPE ligands have been shown to hydrogenate several types of olefinic substrates, such as enamides, with very high enantioselectivity [Burk et al., *J. Am. Chem. Soc*., **1993**, *115*, 10125], while other substrates such as α,β-unsaturated carboxylic acids and allylic alcohols are reduced with only very low selectivities. For example, both β-substituted and β,β-disubstituted α-enamide esters may be hydrogenated to α-amino acid derivatives with high enantioselectivity using certain DuPHOS and BPE-rhodium catalysts [Burk et al., *J. Am. Chem. Soc*., **1995,** *117*, 9375]. Furthermore, β-substituted α-arylenamides may be hydrogenated to α-arylalkylamine derivatives with high enantioselectivities [Burk et al., *J. Am. Chem. Soc*., **1996,** *118*, 5142], yet β,β-disubstituted α-arylenamides are hydrogenated with the same catalysts with very low enantioselectivity (0-5% ee).

One embodiment of the present invention is a complete process for the preparation of 2-substituted succinate derivatives (2) from itaconate substrates 7 and 8, which are easily accessed via the Stobbe condensation. This process involves highly enantioselective hydrogenation of itaconate substrates **7** and **8,** together as an *E*/*Z* mixture or independently, and in the presence or absence of isomerised compound 9. For the hydrogenation, either cationic or neutral rhodium complexes containing the above mentioned chiral phosphine ligands **11-15** may be employed. Apparently, if the ligand is ofthe bisphospholane type, of formula 14 or 15, it is more likely that, if it can be formed, isomerised product **9** will not be hydrogenated.

Also embodied within the invention is a product isolation and purification protocol, whereby the crude hydrogenation product may be conveniently separated from remaining isomerised compound **9**. This protocol entails conversion of the isomerised compound **9** into a more readily separable compound. An example of this method involves treatment of the hydrogenation product mixture, composed of desired product **2** and isomerised compound **9**, with a basic iodine solution, leading to iodolactonisation of **9** while leaving **2** unreacted. Basic extractive procedures allow facile separation of the desired product **2** (remains in basic aqueous phase) and remaining impurities (e.g., neutral iodolactonised products of **9**) and allows isolation of the 2-substituted succinate in a very pure form. Yield and enantiomeric excess of the product **2** are not affected by this procedure. Further purification of the desired 2-substituted succinate **2** may be accomplished through formation of salts (i.e., amine salt formation through addition of amines to carboxylic acid **2**) and subsequent recrystallisation.

The use of a precursor salt is particularly preferred in the case where the hydrogenation substrate is a β,β-disubstituted itaconate derivative, for example wherein R³ =R⁴=methyl. Otherwise, it may be that high substrate conversion is difficult to achieve at acceptable S/C ratios (typically >200:1). See, for example, Examples 18 and 19. In the former, hydrogenation of the *tert*-butylamine salt of 2-isopropylidenesuccinic acid 1-methyl ester, catalysed by a rhodium(I) complex of (*R*,*R*)-methyl BPE, with S/C = 500:1, was conducted at 0°C using methanol as solvent. This gave complete substrate conversion after 20 hours, to afford after salt cracking (*R*)-2-isopropylsuccinic acid 1-methyl ester in 95% ee. Enrichment of the salt to at least 99% ee could then be simply achieved by reslunying in fresh solvent and then filtering, In Example 19, reaction of the free acid of of 2-isopropylidenesuccinic acid 1-methyl ester under similar conditions, with a higher catalyst loading (S/C = 300:1), gave only 33% substrate conversion, with (*R*)-2-isopropylsuccinic acid 1-methyl ester produced in 88% ee.

Overall, the present invention provides a straightforward process for the synthesis of valuable, highly enantiomerically enriched 2-substituted succinates, starting from readily available, inexpensive starting materials.

The following Examples illustrate the invention. Example 17 also illustrates removal of unwanted regioisomer. Preparations 1 to 7 illustrate the preparation of starting materials.
TBME = *tert*-butyl methyl ether
GC = gas chromatographic analysis

### Preparation 1 2-Isobutylidenesuccinic acid mono-methyl ester

Potassium *tert*-butoxide (988 g, 8.8 mol) was dissolved in 6 L *tert*-butanol. To this solution was added a mixture of 577 g (8 mol) isobutyraldehyde and 1.46 kg (10 mol). dimethyl succinate in 1 L *tert*-butanol over a period of 1 h. The mixture was heated to 50°C for 2 h and stirring was continued at room temperature overnight. The solvent was removed on a rotary evaporator, the residue dissolved in 5 L water and extracted twice with 1 L ethyl acetate. The aqueous phase was acidified with concentrated hydrochloric acid and the organic layer was separated. The aqueous phase was extracted twice with 2 L ethyl acetate. The combined organic phases were dried over magnesium sulfate, filtered and the solvent was removed under reduced pressure, yielding 1.468 kg (98 %) of a yellow oil. ¹H-NMR indicated ∼ 60 % of the *E*-isomer, ∼ 13 % *Z*-isomer, ∼19 % isomerised material and ∼ 8 % of another olefinic compound.

Treatment of this crude material with 2 L pentane induced crystallisation. The crystals were filtered off, washed twice with 300 ml pentane and dried *in vacuo.* 658 g (44%) of a light yellow, sticky solid was obtained. ¹H-NMR indicated - 82 % of the E-isomer, ∼7 % Z-isomer, ∼11 % isomerised material.

### Preparation 2 2-Isobutylidenesuccinic acid mono-ethyl ester

To a solution of 11.2 g (0.1 mol) potassium *tert*-butoxide in 100 ml *tert*-butanol was added a mixture of 17.4 g (0.1 mol) diethyl succinate and 7.2 g (0.1 mol) isobutyraldehyde over a period of 30 min. The mixture was allowed to stir at room temperature for one hour and heated to 50°C for an additional hour. The solvent was removed under reduced pressure, the residue was dissolved in 100 ml water and transferred into a separation funnel. The solution was extracted twice with 50 ml ethyl acetate to remove neutral impurities, acidified with 6M HCI and extracted again twice with 20 ml of ethyl acetate. The combined organic phases were dried over magnesium sulfate, filtered and concentrated under reduced pressure, to give 13.6 g (68 %) of a yellow brown oil. ¹H-NMR: 59 % E-isomer, 19 % Z-isomer, 22 % isomerised material.

### Preparation 3 2-(Cyclohexylmethylidene)succinic acid mono-ethyl ester

To a solution of 11.2 g (0.1 mol) potassium *tert*-butoxide in 100 ml tert-butanol was added a mixture of 17.4 g (0.1 mol) diethyl succinate and 11.2 g (0.1 mol) cyclohexylcarboxaldehyde over a period of 30 min. The mixture was allowed to stir at room temperature for one hour and heated to 50°C for an additional hour. The solvent was removed under reduced pressure, the residue was dissolved in 100 ml water and transferred into a separation funnel. The solution was extracted twice with 50 ml ethyl acetate to remove neutral impurities, acidified with 6M HCl and extracted again twice with 20 ml of ethyl acetate. The combined organic phases were dried over magnesium sulfate, filtered and concentrated under reduced pressure, to give 18.9 g (79 %) of a yellow brown oil. ¹H-NMR: 66 % E-isomer, 13 % Z-isomer, 15 *%* cyclohexenyl-isomer, 6 % other unsaturated olefefinic compound.

### Preparation 4 2-(2,2,2-Trimethylethylidene)succinic acid mono-methyl ester

To a solution of 11.2 g (0.1 mol) potassium *tert*-butoxide in 100 ml *tert*-butanol was added a mixture of 14.6 g (0.1 mol) dimethyl succinate and 8.6 g (0.1 mol) trimethylacetaldehyde over a period of 30 min. The mixture was allowed to stir at room temperature for one hour and heated to 50°C for an additional hour. The solvent was removed under reduced pressure, the residue was dissolved in 100 ml water and transferred into a separation funnel. The solution was extracted twice with 50 ml ethyl acetate to remove neutral impurities, acidified with 6M HCl and extracted again twice with 20 ml of ethyl acetate. The combined organic phases were dried over magnesium sulfate, filtered and concentrated under reduced pressure, to give 14.9 g (75 %) of a light yellow creamy solid. ¹H-NMR: > 95 % E-isomer.

### Preparation 5 2-Isopropylidenesuccinic acid mono-ethyl ester

To a solution of 11.2 g (0.1 mol) potassium *tert*-butoxide in 100 ml *tert*-butanol was added a mixture of 17.4 g (0.1 mol) diethyl succinate and 5.6 g (0.1 mol) acetone over a period of 5 min. The mixture was allowed to stir at room temperature for 10 minutes. The solvent was removed under reduced pressure, the residue was dissolved in 100 ml water and transferred into a separation funnel. The solution was extracted twice with 50 ml ethyl acetate to remove neutral impurities, acidified with 6M HCI and extracted again twice with 20 ml of ethyl acetate. The combined organic phases were dried over magnesium sulfate, filtered and concentrated under reduced pressure, to give 15.8 g (85 %) of a brown oil. ¹H-NMR: 86 % conjugated, 14 % deconjugated product.

### Preparation 6 2-Cyclohexylidenesuccinic acid mono-ethyl ester

To a solution of of 17.4 g (0.1 mol) diethyl succinate and 9.8 g (0.1 mol) cyclohexanone in 100 ml *tert*-butanol was added 11.2 g (0.1 mol) potassium *tert*-butoxide in one portion. The mixture was allowed to stir at room temperature for 30 min. 10 ml of water were added and the solvent was removed under reduced pressure. The residue was dissolved in 100 ml water and transferred into a separation funnel. The solution was extracted twice with 50 ml ethyl acetate to remove neutral impurities, acidified with 6M HCl and extracted again twice with 20 ml of ethyl acetate. The combined organic phases were dried over magnesium sulfate, filtered and concentrated under reduced pressure to give 18.5 g (82 %) of a thick yellow oil. ¹H-NMR: 86 % conjugated, 14 % deconjugated product.

### Examples 1 to 16

A solution of 4 mmol of the crude product of one of Preparations 1 to 6, and 3 mmol sodium methanolate in 7 ml degassed methanol, was transferred into a nitrogen-purged 25 ml bomb. The bomb was pressurised three times with 5 atm of hydrogen and subsequently the catalyst (solution in 1 ml of methanol) was injected. The mixture was allowed to stir at room temperature, under a hydrogen pressure of 503 kPa (73 psi) in Examples 1 to 12, 310 kPa (45 psi) in Example 13 and 1034 kPa (150 psi) in Examples 14 to 16. The S:C ratio was 1000:1 in Examples 1 to 12, and 500:1 in Examples 13 to 16.

After the reaction, the pressure was released and the solvent was removed *in vacuo.* The residue was treated with 10 ml 1 M hydrochloric acid and the product was extracted three times with 5 ml dichloromethane. The combined organic phases were dried over magnesium sulfate, filtered and the solvent was removed *in vacuo.* Enantiomeric excess was determined by GC or HPLC using a chiral stationary phase.

Results are shown in Table 1. Conversion was determined based on E- and Z-isomers. n.d. = not determined. In all cases examined, the R,R catalyst afforded (*S*)-2-alkylsuccinate, while S,S catalyst furnished the R isomer.

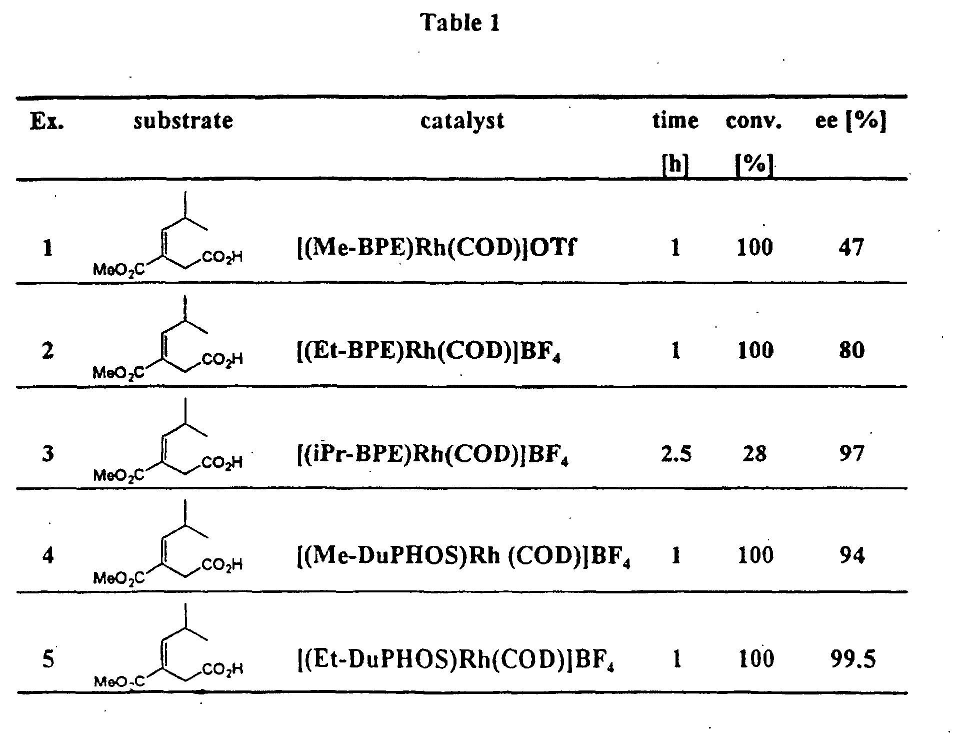

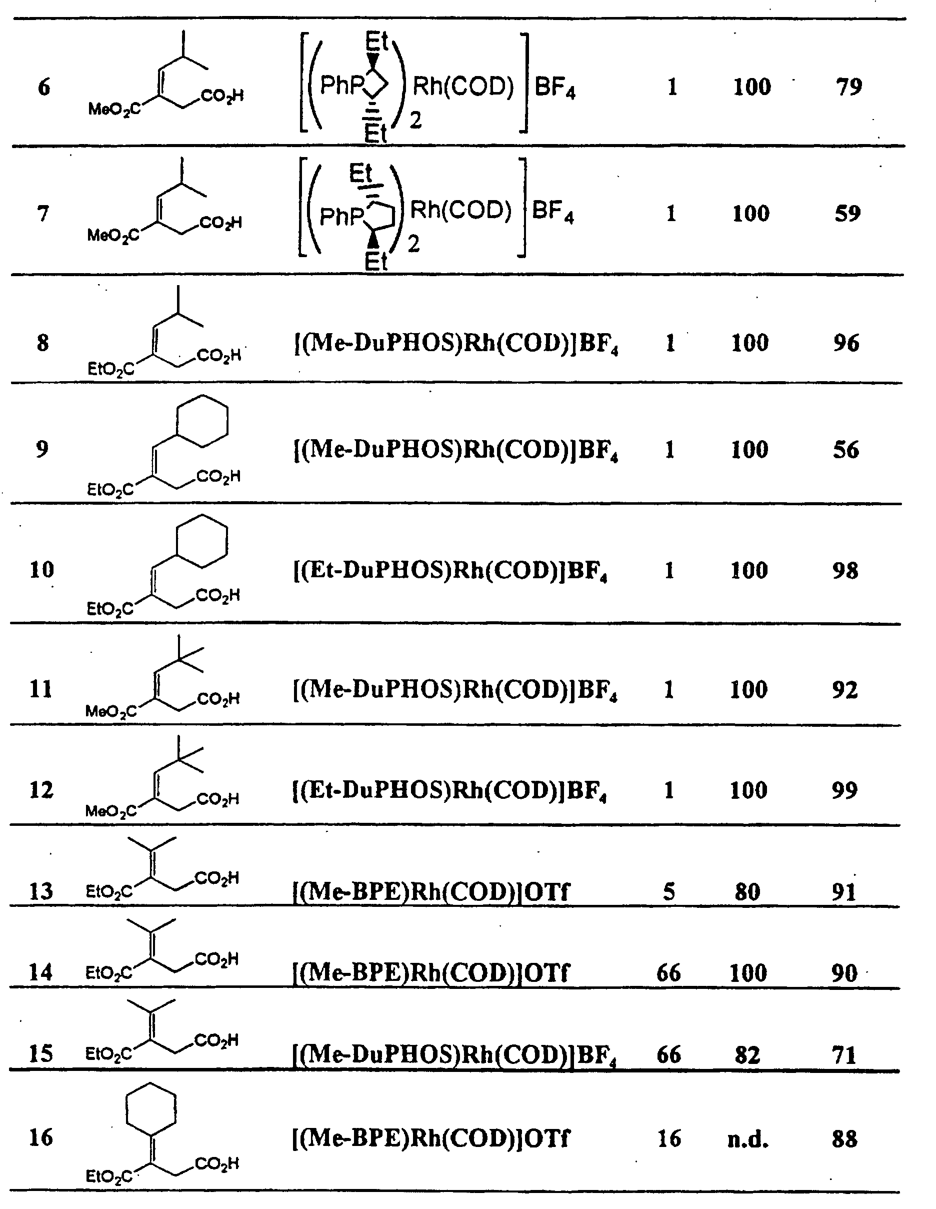

### Example 17

### Asymmetric hydrogenation

To a solution of 532 g (2.86 mol) 2-isobutylenesuccinic acid monomethyl ester (82.7:11 mixture from Preparation 1) in 4.5 L methanol was added 96 g (1.72 mol) sodium methoxide. The mixture was transferred into a 7 L hydrogenation vessel. The mixture was degassed with nitrogen for 2 h and after this pressurised five times with 517 kPa (75 psi) hydrogen under stirring (5 min each). A solution of 540 mg (0.89 mmol) [((R)-MeDuPHOS)Rh(COD)]BF₄ in 20 ml methanol was added and the mixture was allowed to stir under a hydrogen pressure of 200-520 kPa (30-75 psi) for 8 h. NMR analysis revealed that both *E*- and *Z*-isomers were hydrogenated and that the 2-isobutenylsuccinic acid monomethyl ester remained unaffected. The enantiomeric excess of the crude 2-isobutylsuccinic acid monomethyl ester was determined to be 94%.

### Purification by iodolactonisation

The reaction mixture was concentrated to a volume of ∼1 L; 500 ml water, 101 g (1.2 mol) sodium hydrogen carbonate and 119 g (0.47 mol) iodine were added and the mixture was allowed to stir at room temperature for 16 h. Solid sodium sulfite was added to the mixture in order to destroy the excess iodine. The mixture was concentrated on a rotary evaporator to a volume of ∼ 1 L and extracted three times with 0.5 L ethyl acetate. The aqueous phase was acidified with concentrated hydrochloric acid and again extracted three times with 300 ml ethyl acetate. Analysis of the combined organic layers showed only 2-isobutylsuccinic acid monomethyl ester, 94 % ee.

### Crystallisation

The solution above was diluted with 1 L ethyl acetate, and a solution of 219 g (3 mol) *tert*-butylamine in 200 ml ethyl acetate was added over a period of 2 h. The very thick mixture was allowed to stir for an additional 2 h at room temperature. The precipitated solid was filtered, washed three times with 2 L ethyl acetate and dried *in vacuo*, to give 465 g (71 % yield) white powder; > 99 % ee.

### Preparation 7 tert-butylamine salt of 2-isopropylidene succinic acid 1-methyl ester

A solution of *tert*-butylamine (124 mL, 1.19 mol) in *tert*-butyl methyl ether (TBME; 100 mL) was added dropwise, at room temperature, over a period of 2 hours, to a solution of 2-isopropylidenesuccinic acid 1-methyl ester (205 g, 1.19 mol) in TBME (350 mL). The resulting thick suspension was stirred at room temperature for an additional hour, then the solid precipitate was collected, washed with TBME (1 L) and dried under vacuum at 40°C for 48 hours to give 181 g of the salt as a white powder (yield: 62%).

### Example 18

A solution of the *tert*-butylamine salt of 2-isopropylidenesuccinic acid 1-methyl ester (162 g, 0.66 mol) in methanol (800 mL) was transferred to a 2 L high pressure hydrogenation vessel and degassed by pressurizing and venting four times with 10 bar of hydrogen. The vessel was then cooled to 0°C and a solution of [Rh(COD)(*S,S*)-Me-BPE]OTf(0.80 g, 0.0013 mol) in methanol (10 mL) was added through the solvent port. The reaction was purged again with hydrogen and stirred at 0°C under a pressure of hydrogen of 10-7 bar. After 22 hours, the temperature was allowed to raise to room temperature, the vessel was vented in a fume hood, the reaction mixture was transferred to a round-bottomed flask and the solvent was evaporated under reduced pressure. A sample (1 g) of the resulting solid residue was partitioned between HCl 2N (5 mL) and ethyl acetate (5 mL). The organic layer was dried over MgSO₄ and evaporated to give 2-(*S*)-isopropylsuccinic acid 1-methyl ester, ee 96% by GC. The bulk residue was suspended in ethyl acetate (600 mL) and stirred at room temperature for 48 hours, then collected and dried under vacuum to give 154 g of the *tert*-butylamine salt of 2-(*S*)-isopropylsuccinic acid monomethyl ester (yield: 94%). A sample (1 g) of the salt was worked up and analyzed as above, indicating an enantiomeric excess of 99% for the free acid.

### Example 19

2-Isopropylidenesuccinic acid 1-methyl ester (0.86 g, 5.0 mmol) and sodium methoxide (0.10 g, 1.8 mmol) were placed in a 60 mL high pressure hydrogenation vessel and the vessel was purged with hydrogen (by pressurizing and venting three times with 10 bar of hydrogen). Methanol (9 mL, previously degassed by bubbling nitrogen for one hour at room temperature under stirring) was added through the solvent port and the vessel was then cooled to 0°C. A solution of [Rh(COD)(*R,R*)Me-BPE]OTf (0.010 g, 0.016 mmol, substrate/catalyst: 300/1) in methanol (1 mL) was added and the reactor was charged with 10 bar of hydrogen. The reaction was stirred at 0°C for 20 hours, then the solvent was evaporated under reduced pressure and the residue was partitioned between HCl 2N (20 mL) and ethyl acetate (20 mL). The organic layer was separated, dried over MgSO₄, evaporated to give a pale yellow oil. ¹H NMR analysis of the crude indicated that the reduced product and the starting material were present in a ratio 33:67. The enantiomeric excess of2-(*R*)-isopropylsuccinic acid 1-methyl ester was 88% by GC. This result shows that, for this particular substrate, the salt form used in Example 18 is preferable.

### Example 20

The *tert*-butyl ammonium salt of 2-isopropylidenesuccinic acid 1-methyl ester (0.80 g, 3.3 mmol) was placed in a 60 mL high pressure hydrogenation vessel and the vessel was purged with hydrogen (by pressurizing and venting three times with 10 bar of hydrogen). Methanol (9 mL, previously degassed by bubbling nitrogen for one hour at room temperature under stirring) was added through the solvent port and the vessel was then cooled to 0°C. A solution of [Rh(COD)(*R,R*)Me-BPE]OTf (0.004 g, 0.0065 mmol, substrate/catalyst: 500/1) in methanol (1 mL) was added and the reactor was charged with 10 bar of hydrogen. The reaction was stirred at 0°C for 20 hours, then the solvent was evaporated under reduced pressure and the residue was partitioned between HCl 2N (20 mL) and ethyl acetate (20 mL). The organic layer was separated, dried over MgSO₄, evaporated to give a pale yellow oil. ¹H NMR analysis of the crude indicated that the conversion to the reduction product was more than 95%. The enantiomeric excess of 2-(*R*)-isopropylsuccinic acid 1-methyl ester was 95% by GC.

### Example 23

The *tert*-butylamine salt of (*E*)-2-(3-phenyl-2-propenylidene)succinic acid 1-methyl ester (1 g, 4.1 mmol) and [Rh(COD)(*R,R*)Me-DuPhos]BF₄ (6 mg, 0.01 mmol, substrate/catalyst: 400:1) were weighed in a 60 mL high pressure hydrogenation vessel and an atmosphere of nitrogen was introduced by evacuating the reactor and refilling with oxygen-free dry nitrogen. This procedure was repeated three times. Methanol (5 mL, previously degassed by bubbling nitrogen for one hour at room temperature while stirring) was added to the reactor through the solvent port. The reactor was charged with 690 kPa (100 psi) of hydrogen and the pressure released. The reactor was then repressurised to 965 kPa (140 psi) and the reaction was stirred for 16 hours, then the solvent was evaporate under reduced pressure and the residue was partitioned between HCl 2N (20 mL) and ethyl acetate (20 mL). The organic layer was separated, dried over MgSO₄, evaporated to give a pale yellow oil. ¹H NMR analysis of the crude indicated that the conversion to the reduction product was complete. The enantiomeric excess of 2-(S)-(3-phenyl-2-propenyl)succinic acid 1-methyl ester was 99% by GC.

### Example 22

The *tert*-butylamine salt of 2-cyclohexylidenesuccinic acid 1-methyl ester (0.91 g, 3.2 mmol) was placed in a 60 mL high pressure hydrogenation vessel and the vessel was purged with hydrogen (by pressurizing and venting three times with 10 bar of hydrogen). Methanol (9 mL, previously degassed by bubbling nitrogen for one hour at room temperature under stirring) was added through the solvent port and the vessel was then cooled to 0°C. A solution of [Rh(COD)(R,R)Me-BPE]OTf (0.004 g, 0.0065 mmol, substrate/catalyst: 500/1) in methanol (1 mL) was added and the reactor was charged with 10 bar of hydrogen. The reaction was stirred at 0°C for 20 hours, then the solvent was evaporated under reduced pressure and the residue was partitioned between HCl 2N (20 mL) and ethyl acetate (20 mL). The organic layer was separated, dried over MgSO₄, evaporated to give 0.75 g of 2-cyclohexylsuccinic acid monomethyl ester as a pale yellow oil (yield 82%). The enantiomeric excess of 2-(R)-cyclohexylsuccinic acid 1-methyl ester was 96% by GC.

### Example 23

The *tert*-butylamine salt of2-(2-adamantylidene)succinic acid 1-methyl ester (0.54 g, 1.6 mmol) was placed in a 60 mL high pressure hydrogenation vessel and the vessel was purged with hydrogen (by pressurizing and venting three times with 10 bar of hydrogen). Methanol (9 mL, previously degassed by bubbling nitrogen for one hour at room temperature under stirring) was added through the solvent port and the vessel was then cooled to 0°C. A solution of [Rh(COD)(R,R)Me-BPE]OTf (0.004 g, 0.0065 mmol, substrate/catalyst: ∼250/1) in methanol (1 mL) was added and the reactor was charged with 10 bar of hydrogen. The reaction was stirred at 0°C for 23 hours, then the solvent was evaporated under reduced pressure and the residue was partitioned between HCl 2N (20 mL) and ethyl acetate (20 mL). The organic layer was separated, dried over MgSO₄, evaporated to give a pale yellow oil. ¹H NMR analysis of the crude indicated that the conversion to the reduction product was complete. The enantiomeric excess of 2-(*R*)-(2-adamantanyl)succinic acid monomethyl ester was 78% by GC. In similar experiments, carried out at room temperature (approx. 20°C), 2-(*R*)-(2-adamantanyl)succinic acid monomethyl ester was obtained with 63% ee.

## Claims

1. A process for the preparation of an enantiomerically enriched 2-substituted succinic acid derivative of formula **2**, which comprises asymmetric hydrogenation of a dehydro precursor of formula **7** or **8**, or a mixture thereof 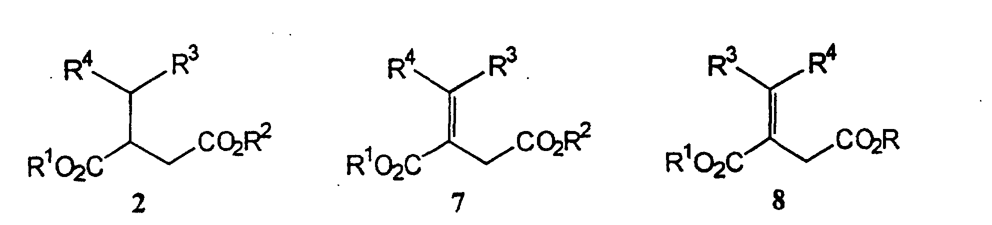 wherein R¹ and R² are each independently H, a salt-forming cation or an organic group of up to 30 C atoms, and R³ and R⁴ are each independently H or an organic group of up to 30 C atoms, or are linked to form a ring, provided that R³ and R⁴ are not both H, in the presence of a transition metal complex of a chiral phosphine ligand having the partial formula **10** 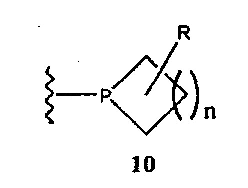 wherein n is 0 to 6 and R represents at least one non-hydrogen organic group of up to 30 C atoms.

2. A process for the preparation of an enantiomerically enriched 2-substituted succinic acid derivative of formula 2, as defined in claim 1, which comprises asymmetric hydrogenation of a precursor as defined in claim 1, or a mixture thereof, wherein the precursor is a salt.

3. A process for the preparation of an enantiomerically enriched 2-substituted succinic acid derivative of formula **2,** as defined in claim 1, which comprises asymmetric hydrogenation of a precursor as defined in claim 1, or a mixture thereof, wherein R⁴ is CHR⁵R⁶, R⁵ and R⁶ each being H or any other group consistent with the definition of R⁴, in the presence of a transition metal complex of a chiral phosphine ligand; conversion of the isomerised (deconjugated) compound 9 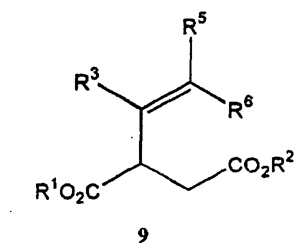 into a compound readily separable from the hydrogenation product 2; and removal of said separable compound.

4. A process according to claim 3, wherein the conversion comprises treatment of the isomerised substrate **9** with basic iodine, to give a lactone derivative, and the removal comprises extraction.

5. A process according to any of claims 1, 3 and 4, wherein the precursor is a salt.

6. A process according to claim 2 or claim 5, wherein the precursor is an amine or phosphine salt.

7. A process according to claim 6, wherein the precursor is an amine salt.

8. A process according to claim 7, wherein the amine is a primary amine.

9. A process according to claim 8, wherein the amine is *tert*-butylamine.

10. A process according to claim 7, wherein the amine is a tertiary amine.

11. A process according to claim 10, wherein the amine is triethylamine.

12. A process according to any of claims 6 to 11, wherein the precursor salt is isolated prior to use in the process.

13. A process according to claim 2 or claim 5, wherein a metal salt of the precursor is generated *in situ.*

14. A process according to any of claims 2 to 13, wherein the ligand is as defined in claim 1.

15. A process according to any preceding claim, wherein the ligand is of formula **11** or 12 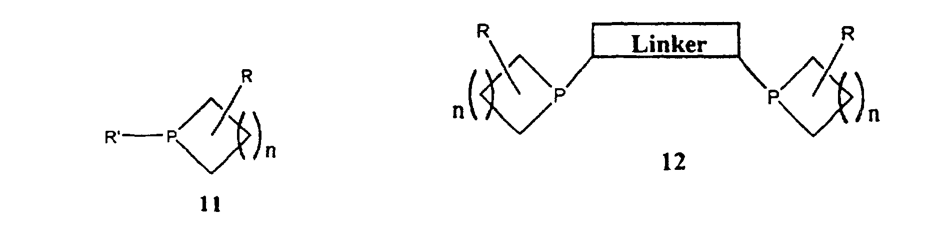 wherein Linker and R' are independently any non-hydrogen organic group of up to 30 C atoms.

16. A process according to claim 15, wherein the ligand is of formula 14 or 15

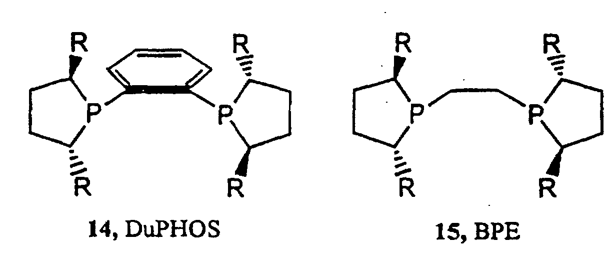

17. A process according to claim 16, wherein Ris a C₁₋₈ linear or branched alkyl group, or an aromatic group.

18. A process according to any preceding claim, wherein R³ and R⁴ are each, or linked to form, an organic group of up to 30 C atoms.

19. A process according to claim 18, wherein R³=R⁴.

20. A process according to any of claims 1 to 17, wherein one of R³ and R⁴ is H.

21. A process according to any preceding claim, wherein R³ and R⁴ are independently H, C₁₋₂₀ alkyl or aralkyl.

22. A process according to any preceding claim, wherein R² is H or a cation.

23. A process according to claim 22, wherein R¹ is C₁₋₁₀ alkyl or aralkyl.

24. A process according to any preceding claim, wherein the transition metal is rhodium.

25. A process according to any preceding claim, wherein the reaction temperature is less than 10°C.

26. A process according to any preceding claim, which gives the product **2** in an enantiomeric excess of at least 90%.

## Revendications

1. Procédé de préparation d'un dérivé d'acide succinique 2-substitué énantiomériquement enrichi de formule 2, comprenant l'hydrogénation asymétrique d'un précurseur déshydro de formule 7 ou 8, ou d'un mélange de ceux-ci 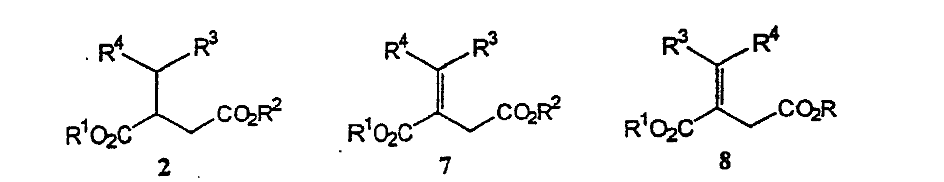 dans lesquelles R¹ et R² sont chacun indépendamment H, un cation formant un sel ou un groupe organique ayant jusqu'à 30 atomes de carbone, et R³ et R⁴ sont chacun indépendamment H ou un groupe organique ayant jusqu'à 30 atomes de carbone, ou sont liés pour former un cycle, à condition que R³ et R⁴ ne soient pas tous les deux H, en présence d'un complexe de métal de transition d'un ligand phosphine chiral de formule partielle 10 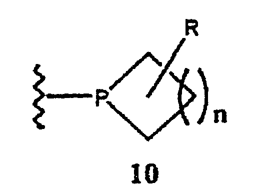 dans laquelle n vaut de 0 à 6 et R représente au moins un groupe organique différent d'un hydrogène ayant jusqu'à 30 atomes de carbone.

2. Procédé de préparation d'un dérivé d'acide succinique 2-substitué énantiomériquement enrichi de formule 2, tel que défini dans la revendication 1, comprenant l'hydrogénation asymétrique d'un précurseur tel que défini dans la revendication 1, ou d'un mélange de ceux-ci, dans lequel le précurseur est un sel.

3. Procédé de préparation d'un dérivé d'acide succinique 2-substitué énantiomériquement enrichi de formule 2, tel que défini dans la revendication 1, comprenant l'hydrogénation asymétrique d'un précurseur tel que défini dans la revendication 1, ou d'un mélange de ceux-ci, dans laquelle R⁴ est CHR⁵R⁶, R⁵ et R⁶ étant chacun H ou tout autre groupe cohérent avec la définition de R⁴, en présence d'un complexe de métal de transition d'un ligand phosphine chiral ; la transformation du composé isomérisé (déconjugué) 9 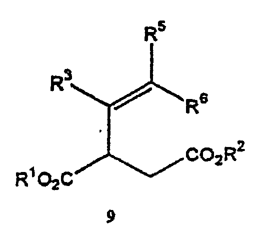 en un composé aisément séparable du produit d'hydrogénation 2 ; et l'élimination dudit composé séparable.

4. Procédé selon la revendication 3, dans lequel la transformation comprend le traitement du substrat isomérisé 9 avec de l'iode basique, pour donner lieu à un dérivé lactone, et l'élimination comprend l'extraction.

5. Procédé selon l'une quelconque des revendications 1, 3 et 4, dans lequel le précurseur est un sel.

6. Procédé selon la revendication 2 ou la revendication 5, dans lequel le précurseur est un sel d'amine ou de phosphine.

7. Procédé selon la revendication 6, dans lequel le précurseur est un sel d'amine.

8. Procédé selon la revendication 7, dans lequel l'amine est une amine primaire.

9. Procédé selon la revendication 8, dans lequel l'amine est la *tert*-butylamine.

10. Procédé selon la revendication 7, dans lequel l'amine est une amine tertiaire.

11. Procédé selon la revendication 10, dans lequel l'amine est la triéthylamine.

12. Procédé selon l'une quelconque des revendications 6 à 11, dans lequel le sel précurseur est isolé avant l'utilisation dans le procédé.

13. Procédé selon la revendication 2 ou la revendication 5, dans lequel un sel métallique du précurseur est généré *in situ.*

14. Procédé selon l'une quelconque des revendications 2 à 13, dans lequel le ligand est tel que défini dans la revendication 1.

15. Procédé selon l'une quelconque des revendications précédentes, dans lequel le ligand est de formules 11 ou 12 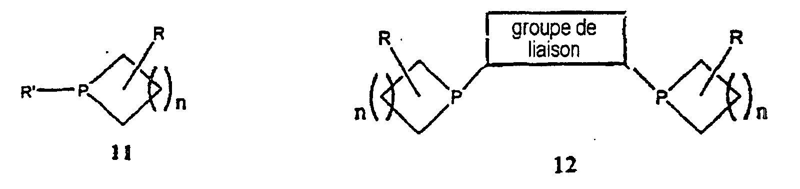 dans lequel le groupe de liaison et R' sont indépendamment tout groupe organique différent d'un hydrogène ayant jusqu'à 30 atomes de carbone.

16. Procédé selon la revendication 15, dans lequel le ligand est de formule 14 ou 15

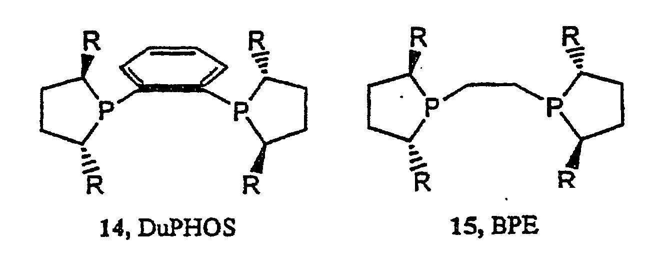

17. Procédé selon la revendication 16, dans lequel R est un groupe alkyle linéaire ou ramifié en C₁₋₈ ou un groupe aromatique.

18. Procédé selon l'une quelconque des revendications précédentes, dans lequel R³ et R⁴ sont chacun, ou liés pour former, un groupe organique ayant jusqu'à 30 atomes de carbone.

19. Procédé selon la revendication 18, dans lequel R³ = R⁴.

20. Procédé selon l'une quelconque des revendications 1 à 17, dans lequel l'un parmi R³ et R⁴ est H.

21. Procédé selon l'une quelconque des revendications précédentes, dans lequel R³ et R⁴ sont indépendamment H, un alkyle ou un aralkyle en C₁₋₂₀.

22. Procédé selon l'une quelconque des revendications précédentes, dans lequel R² est H ou un cation.

23. Procédé selon la revendication 22, dans lequel R¹ est un alkyle ou un aralkyle en C₁₋₁₀.

24. Procédé selon l'une quelconque des revendications précédentes, dans lequel le métal de transition est le rhodium.

25. Procédé selon l'une quelconque des revendications précédentes, dans lequel la température de réaction est inférieure à 10°C.

26. Procédé selon l'une quelconque des revendications précédentes, donnant lieu au produit 2 avec un excès énantiomérique d'au moins 90 %.

## Patentansprüche

1. Verfahren zur Herstellung eines enantiomer angereicherten 2-substituierten Bernsteinsäurederivats der Formel 2, welches umfasst die asymmetrische Hydrierung einer Dehydrovorstufe der Formel 7 oder 8 oder einer Mischung davon 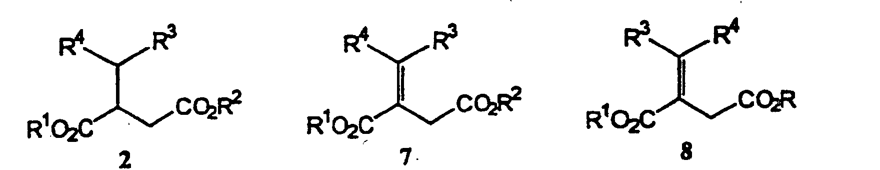 worin R¹ und R² jeweils unabhängig H, ein Salz-bildendes Kation oder eine organische Gruppe mit bis zu 30 C-Atomen sind und R³ und R⁴ jeweils unabhängig H oder eine organische Gruppe mit bis zu 30 C-Atomen sind oder unter Bildung eines Rings verknüpft sind, mit der Maßgabe, dass R³ und R⁴ nicht beide H sind, in Anwesenheit eines Übergangsmetallkomplexes eines chiralen Phosphinliganden mit der Teilformel 10 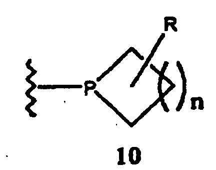 worin n 0 bis 6 ist und R mindestens eine organische Gruppe mit bis zu 30 C-Atomen ist, die nicht Wasserstoff ist.

2. Verfahren zur Herstellung eines enantiomer angereicherten 2-substituierten Bernsteinsäurederivats der Formel 2 wie in Anspruch 1 definiert, welches die asymmetrische Hydrierung einer Vorstufe wie in Anspruch 1 definiert oder einer Mischung davon umfasst, wobei die Vorstufe ein Salz ist.

3. Verfahren zur Herstellung eines enantiomer angereicherten 2-substituierten Bernsteinsäurederivats der Formel 2 wie in Anspruch 1 definiert, welches umfasst die asymmetrische Hydrierung einer Vorstufe wie in Anspruch 1 definiert oder einer Mischung davon, wobei R⁴ CHR⁵R⁶ ist, worin R⁵ und R⁶ jeweils H oder irgendeine andere Gruppe, die mit der Definition von R⁴ in Einklang steht, sind, in Anwesenheit eines Übergangsmetallkomplexes eines chiralen Phosphinliganden, die Umwandlung der isomerisierten (dekonjugierten) Verbindung 9 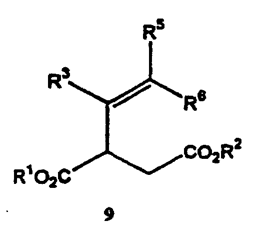 in eine Verbindung, die ohne weiteres vom Hydrierungsprodukt 2 abtrennbar ist, und die Entfernung der genannten abtrennbaren Verbindung.

4. Verfahren nach Anspruch 3, worin die Umwandlung die Behandlung des isomerisierten Substrats 9 mit basischem lod unter Bildung eines Lactonderivats umfasst und die Entfernung eine Extraktion umfasst.

5. Verfahren nach irgendeinem der Ansprüche 1, 3 und 4, worin die Vorstufe ein Salz ist.

6. Verfahren nach Anspruch 2 oder Anspruch 5, worin die Vorstufe ein Aminoder Phosphinsalz ist.

7. Verfahren nach Anspruch 6, worin die Vorstufe ein Aminsalz ist.

8. Verfahren nach Anspruch 7, worin das Amin ein primäres Amin ist.

9. Verfahren nach Anspruch 8, worin das Amin tert.-Butylamin ist.

10. Verfahren nach Anspruch 7, worin das Amin ein tertiäres Amin ist.

11. Verfahren nach Anspruch 10, worin das Amin Triethylamin ist.

12. Verfahren nach irgendeinem der Ansprüche 6 bis 11, worin das Vorstufensalz vor dem Einsatz im Verfahren isoliert wird.

13. Verfahren nach Anspruch 2 oder Anspruch 5, worin ein Metallsalz der Vorstufe in situ erzeugt wird.

14. Verfahren nach irgendeinem der Ansprüche 2 bis 13, worin der Ligand wie in Anspruch 1 definiert ist.

15. Verfahren nach irgendeinem vorhergehenden Anspruch, worin der Ligand die Formel 11 oder 12 aufweist 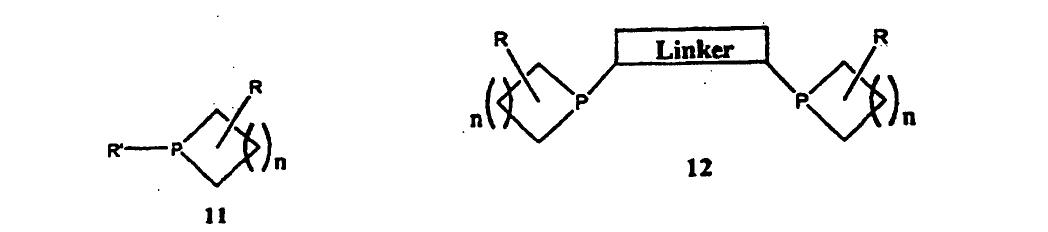 wobei der Linker und R' unabhängig irgendeine organische Gruppe mit bis zu 30 C-Atomen sind, die nicht Wasserstoff ist.

16. Verfahren nach Anspruch 15, worin der Ligand die Formel 14 oder 15 aufweist

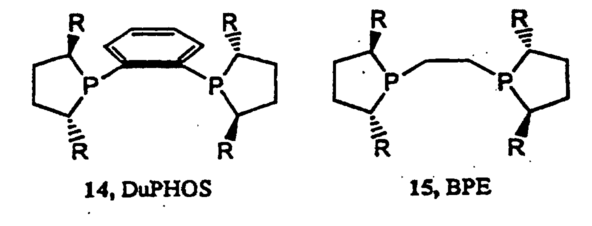

17. Verfahren nach Anspruch 16, worin R eine lineare oder verzweigte C₁₋₈-Alkylgruppe oder eine aromatische Gruppe ist.

18. Verfahren nach irgendeinem vorhergehenden Anspruch, worin R³ und R⁴ jeweils eine organische Gruppe mit bis zu 30 C-Atomen sind oder unter Bildung dieser Gruppe verknüpft sind.

19. Verfahren nach Anspruch 18, worin R³ = R⁴.

20. Verfahren nach irgendeinem der Ansprüche 1 bis 17, worin eine von R³ und R⁴ H ist.

21. Verfahren nach irgendeinem vorhergehenden Anspruch, worin R³ und R⁴ unabhängig H, C₁₋₂₀-Alkyl oder Aralkyl sind.

22. Verfahren nach irgendeinem vorhergehenden Anspruch, worin R² H oder ein Kation ist.

23. Verfahren nach Anspruch 22, worin R¹ C₁₋₁₀-Alkyl oder Aralkyl ist.

24. Verfahren nach irgendeinem vorhergehenden Anspruch, worin das Übergangsmetall Rhodium ist.

25. Verfahren nach irgendeinem vorhergehenden Anspruch, worin die Reaktionstemperatur weniger als 10°C beträgt.

26. Verfahren nach irgendeinem vorhergehenden Anspruch, welches das Produkt 2 in einem Enantiomerenüberschuss von mindestens 90% ergibt.
